# EUROPEAN PATENT SPECIFICATION

(11) **EP 2 482 831 B1**
(45) Date of publication and mention of the grant of the patent: **28.02.2018**
(21) Application number: 10760745.9
(22) Date of filing: 29.09.2010
(51) Int. Cl.: A61K 36/53, A61K 36/537, A61P 31/16, A61P 11/00

(54) **COMPOSITIONS OF BOTANICAL EXTRACTS AND THEIR USE**
ZUSAMMENSETZUNGEN PFLANZLICHER EXTRAKTE UND DEREN VERWENDUNG
COMPOSITIONS D'EXTRAITS BOTANIQUES ET LEUR UTILISATION

(30) Priority: 29.09.2009 GB 0917086
(43) Date of publication of application: 08.08.2012
(73) Proprietor: Olvos Science S.A., 14564 Athens (GR)
(72) Inventor: CASTANAS, Elias, 713 05 Heraklion (GR); PIRINTSOS, Stergios, 740 57 Rethymno (GR); LIONIS, Christos, 741 00 Rethymno (GR)
(74) Representative: Towler, Philip Dean
(86) International application number: PCT/GB2010/001836
(87) International publication number: WO 2011/045557

(56) References cited:
- WO-A2-2007/075580
- WO-A2-2008/133982
- DATABASE WPI Week 200060 Thomson Scientific, London, GB; AN 2000-621741 XP002617197, & JP 2000 229870 A (BIOGRIP SL) 22 August 2000 (2000-08-22)

## Description

### FIELD OF THE INVENTION

This invention relates generally to the use of botanical extracts for treating, preventing or ameliorating the symptoms of influenza viruses, notably type A/H1N1 species, and coronavirus infection. More specifically, the invention provides compositions of botanical extracts that are suitable for the preparation of pharmaceutically active compositions for combating the aforementioned infections. Methods for preparation and formulation of the compositions are also provided.

### BACKGROUND OF THE INVENTION

Microbial or viral infections of the upper respiratory tract are a common problem in all parts of the world. They cause discomfort, inconvenience and, in extreme cases, severe suffering or even death. Due to their frequency, they also have a significant economic impact due to sickness absence. There is an ongoing need for effective treatments for such ailments.

Novel influenza type A (H1N1), also known as "swine flu", is an influenza virus causing illness in humans. The virus can be passed from person to person by exposure to infected droplets expelled by coughing or sneezing that can be inhaled, or that can contaminate hands or surfaces.

As influenza A (H1N1) is a new virus, most people have little or no immunity to it. Consequently, this virus could cause more infections than are seen with seasonal flu. The new influenza A (H1N1) appears to be as contagious as seasonal influenza, and has spread quickly, particularly among young people (from ages 10 to 45). Symptoms of influenza A (H1N1) are flu-like, including fever, cough, headache, muscle and joint pain, sore throat and runny nose, and sometimes vomiting and diarrhoea. The severity of the disease ranges from very mild symptoms to severe illnesses that can result in death. The majority of people who contract the virus experience the milder disease and recover without antiviral treatment or medical care. Of the more serious cases, more than half of hospitalized people had underlying health conditions or weak immune systems.

Antiviral drugs may reduce the symptoms and duration of illness, just as they do for seasonal influenza. They also may contribute to preventing severe disease and death.

There are two classes of antiviral drugs for influenza: inhibitors of neuraminidase such as oseltamivir and zanamivir; and adamantanes, such as amantadine and rimantadine. Tests on viruses obtained from patients in Mexico and the United States have indicated that current new H1N1 viruses are sensitive to neuraminidase inhibitors, but resistant to the other class, the adamantanes.

Oseltamivir-resistant pandemic (H1N1) 2009 influenza viruses have already been detected in Denmark, Hong Kong SAR, Japan and Canada.

At the public level vaccines are the most powerful tool for control of influenza. Nevertheless, the seasonal flu vaccine is not expected to protect against the novel H1N1 flu, whilst at the time of writing the production and testing of novel H1N1 vaccine is incomplete. Moreover, only certain groups of the population are expected to receive the novel H1N1 vaccine when it first becomes available, so other groups of population will be excluded. In addition, if the vaccine is recommended for use to a group of population, those who choose vaccination for themselves or their children will be screened for contraindications to vaccination (such as an allergy to eggs), and will receive information sheets describing the vaccine's risks and benefits, possible adverse events associated with vaccination, and how to report these events. As a result, some of these are expected to refuse vaccination for themselves or their children, and so more groups of the population will be added to those excluded. Given the above limitations related to both the antiviral drugs for influenza and the novel H1N1 vaccine, a need still exists for treating, preventing or ameliorating the symptoms arising from the flu virus H1N1, as well as other influenza viruses and other microbial or viral infections of the upper respiratory tract, such as those caused by rhinovirus, coronavirus and human respiratory syncytial virus.

A number of studies have been undertaken regarding the pharmaceutical activity of botanical extracts. For example, Loizzo et al, Chemistry & Biodiversity 5: 461-470 (2008) investigated the antiviral activities of the essential oils of seven plant species found in the Lebanon, with particular reference to the SARS coronavirus and *Herpes simplex* virus type 1.

The essential oils are considered to be among the most important antimicrobial agents present in these plants, and may also have antioxidant and anti-inflammatory activities. Volatile oils are a complex mixture of compounds, mainly monoterpenes, sesquiterpenes, and their oxygenated derivatives (alcohols, aldehydes, esters, ethers, ketones, phenols and oxides). Other volatile compounds include phenylpropenes and specific sulphur- or nitrogen-containing substances. For detailed surveys, see Cowan, Clinical Microbiology Reviews 12: 564-582 (1999) and Burt, International Journal of Food Microbiology 94: 223-253 (2004).

It is known that the essential oils obtained from traditional Mediterranean herbs, posses strong antimicrobial activity due to their very high content of monoterpenes and oxygenated compounds, such as γ-terpinene, p-cymene, thymol and carvacrol (Liolios et al., Food Chemistry 112: 77-83 (2009)).

The essential oils are hydrophobic and their primary site of activity is the cell membrane. They accumulate in the lipid bilayer according to a partition coefficient that is specific for the compound applied, leading to disruption of the membrane structure and function (Pol & Smid, Letters in Applied Microbiology, 29: 166-170 (1999)).

The antimicrobial activity of the essential oils has been attributed to their constituent monoterpenes that, due to their lipophilic character, act by disrupting the microbial cytoplasmic membrane, which thus loses its high impermeability for protons and larger ions. When the disturbance of membrane integrity occurs, then its functions are compromised, not only as a barrier but also as a matrix for enzymes and as an energy transducer (Cristiani et al, Journal of Agricultural and Food Chemistry 55: 6300-6308 (2007)).

Additionally, some volatile essential oils of commonly used culinary herbs, spices and herbal teas have also exhibited a high level of antiviral activity. Medicinal plants have a variety of chemical constituents, which have the ability to inhibit the replication cycle of various types of DNA or RNA viruses. Compounds from natural sources are of interest as possible sources to control viral infection. In this context various research groups in Asia, Far East, Europe and America have given particular attention to developing antiviral agents from their native traditional plant medicines, see Jassim and Naji, J. Applied Microbiology 95: 412-427 (2003). According to these authors, in the case of terpenoids (sesquiterpene and triterpenoids) the mechanism of the antiviral effect is related to a membrane-mediated mechanism and inhibition of viral DNA synthesis.

### SUMMARY OF THE INVENTION

The present invention provides novel compositions comprising certain botanical extracts. These compositions are active against H1N1 influenza virus as well as other influenza viruses and other microbial or viral infections of the upper respiratory tract.

Accordingly, in a first aspect the invention provides a composition comprising effective amounts of extracts of the following plants:
(a) *Coridothymus capitatus*;
(b) *Origanum dictamnus*; and
(c) at least one of *Salvia fruticosa* or *Salvia pomifera.*

The said extracts are obtainable by hydro-alcoholic extraction and/or by hydro-distillation of the plant leaves.

In addition to the above active ingredients the compositions may comprise one or more pharmaceutically acceptable carrier, diluent, excipient or adjuvant.

The compositions of the present invention can also be used in conjunction with other therapeutic/protective agents or adjunctive therapies commonly used to treat influenza and other microbial or viral infections of the upper respiratory tract, thus enhancing the therapeutically desired effect.

The extracts are typically used in the following percentages by volume, based on the total volume of the extracts of the species (a), (b) and (c):
(a) *Coridothymus capitatus*: 40-80%, preferably 50-75%, more preferably 57-65%;
(b) *Origanum dictamnus*: 3-15%, preferably 5-12%, more preferably 7-9%.
(c) *Salvia fruticosa* and/or *Salvia pomifera*: 17-45%, preferably 20-38%, more preferably 28-34%.

The most preferred composition comprises therapeutically effective amounts of four parts by volume of the extract of species (a), two parts by volume of the extract of species (c) and half part by volume of the extract of species (b).

While extracts of the species (a), (b) and (c) have been known to demonstrate health benefits when administered individually, the present invention relates to novel combinations of the natural extracts. The combinations are considered to exhibit advantageous properties beyond those expected from the properties of the individual components.

In another aspect, this invention relates to the extracts of species (a), (b) and (c) as defined above for use in preventing or treating influenza or coronavirus infection. Said extracts may be administered simultaneously or sequentially. Simultaneous administration may be effected using a composition of the invention.

In another aspect, this invention relates to a combination of the extracts of species (a), (b) and (c) as defined above, for use in therapy. The combination may take the form of a composition of the invention.

In another aspect, this invention relates to a combination of the extracts of species (a), (b) and (c) as defined above, for use in preventing or treating influenza or coronavirus infection. The combination may take the form of a composition of the invention.

In another aspect, this invention relates to the use of the extracts of species (a), (b) and (c) as defined above, for the manufacture of a medicament for preventing or treating influenza or coronavirus infection.

In another aspect, this invention relates to a process comprising the following steps:
1) providing extracts of species (a), (b) and (c) as defined above; and
2) mixing, in any order, the extracts and optionally one or more pharmaceutically acceptable carrier, diluent, excipient or adjuvant.
If desired, the mixture may be encapsulated or otherwise made into a unit dosage form, thereby to facilitate convenient administration.

A better understanding of the invention will be obtained from the following detailed description of the invention, as set forth and exemplified in the description and illustrative embodiments.

### BRIEF DESCRIPTION OF THE DRAWINGS

Figure 1 shows the ranges for the content of the four main essential components of *Coridothymus capitatus* obtained from 10 different samples.
Figure 2 shows the ranges for the content of carvacrol, p-cymene and γ-terpinene in *Origanum dictamnus* obtained from 14 different samples.
Figure 3 shows the ranges for the content of 1,8-cineole and b-thujone in *Salvia fruticosa* obtained from 12 different samples.
Figure 4 shows the ranges for the content of a-thujone, b-thujone and myrcene in *Salvia pomifera* obtained from 9 different samples.
Figure 5 is a GC-MS output of a hydrodistillate combined extract resulting from a dry plant material mixture of a preferred composition of the invention.
Figure 6 provides an example of the anti-viral capacity of a preferred extract mixture of the invention, showing as bar charts the effect of the extract mixture on Herpes Simplex Virus.
Figure 7 is a graph showing the stability of a hydro-alcoholic preparation of a preferred extract of the invention.
Figure 8 is a graph showing the stability of a preferred extract of the invention in an olive oil vehicle.

### DETAILED DESCRIPTION OF THE INVENTION

The present invention relates to the use of the extracts of species (a) *Coridothymus capitatus,* (b) *Origanum dictamnus,* and (c) *Salvia fruticosa* and/or *Salvia pomifera,* for preventing or treating influenza and coronavirus infection.

The combination of extracts of the species (a), (b) and (c) is especially preferred for the treatment of influenza type A (H1N1).
For a proper characterisation of the said species, the following botanical information is provided:
*Coridothymus capitatus* (L.) Reichenb. fil. [syn. *Thymus capitatus* (L.) Hoffmanns. & Link] is a dwarf-shrub of the Labiatae family which is sometimes referred to as "Spanish Oregano". It is found throughout the Mediterranean region and is the only member of the monospecific genus *Coridothymus.* It is a somewhat aromatic shrub, growing to 50 cm, densely branched with erect or ascending stems having axillary leaf-clusters. The leaves are linear, rather fleshy, and gland-dotted with a flat margin. Flowers are purplish-pink or pink, 7-10 mm long, borne in terminal, rather oblong clusters; bracts are green, often red-tinged, oval, closely overlapping to form a cone-like head; the calyx has 20-22 veins (10-13 in other thymes). The plant grows on limestone terrain, on chalk and marl in mountainous areas, and on the coastal plain. The plant flowers from May to October. It is collected throughout the year.
*Origanum dictamnus [Amaracus dictamnus]* is a densely, white-woolly, tufted dwarf shrub; stems are branched at the base, and grow to 20 cm long. Its leaves are rounded to oval, untoothed, short-stalked, grayish or whitish, with conspicuous raised veins. Flowers are pink, 8-11 mm long, borne in pendent, oblong spikelets, in opposite, stalked pairs, with closely overlapping purple bracts; corolla is long-tubed. The plant grows on rocky places, particularly cliff crevices, generally in shade or semi-shade. The flowering period is June -September. Distribution: It is an endemic plant species of Crete. Local names: in Greek Cretan dialect "Δ 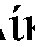 κταµo", or in Cretan dialect 'Eρωντα 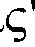 ".
*Salvia fruticosa* is a strongly aromatic, rather grayish shrub growing to 1.2 m with white-felted stems. Its leaves often have 1-2 pairs of small lobes below the main one. Flowers are lilac, pink or sometimes white, 16-25 mm long. In its native environment it grows as part of the maquis shrubland, garique and several other open plant communities, in rocky places and gullies, and at roadsides. It is also grown as an ornamental flowering shrub, preferring full sun, well-draining soil, and good air circulation. Flowering period: March - June. Distribution: Italy, Sicily, and the Balkans eastwards, including Cyprus. Due to its wide variation in leaf shape, there has been a great deal of taxonomic confusion over the years, with many of the leaf variations of *Salvia fruticosa* being named as distinct species. These include *S*. *libanotica, S. triloba, S. lobryana,* and *S. cypria,* which are now considered to be *Salvia fruticosa.* The variation in leaf depends on geographical area. For example, plants growing on the western part of Crete have entire leaves with flat blade and margins and dark green upper sides. Plants growing on the eastern side of the island have much smaller leaves, with deeply three-lobed yellowish-green blade and undulate margins. The variation continues throughout different parts of Greece. Adding to the confusion over the name, the plant has also been called *Salvia triloba,* as named by Carl Linnaeus in 1781, until it was discovered that it was the same as the plant named by Philip Miller in 1768, with the earlier name receiving preference according to plant naming conventions. Local names include sage apple, Khokh barri, and Na'ama Hobeiq'es-sedr.
*Salvia pomifera* is like *Salvia fruticosa,* but its leaves are oval and flowers are violet-blue; its calyx is much enlarged in fruit. Distribution: South Greece and Aegean Islands. According to earlier taxonomists, it was separated into two different species, *S. pomifera* and *S. calycina* Sm. Hedge (1982) (Hedge, I. C., 1982. Salvia L. In: Davis, P. H. (Ed.), Flora of Turkey and the East Aegean islands vol. 7, pp. 400-461. Edinburgh University Press, Edinburgh.) synonymizes these two species under *S. pomifera,* whereas in other approaches they were considered subspecies of *S. pomifera,* viz. subsp. *pomifera* and subsp. *calycina* (Sm.) Hayek (see Hayek, A., 1931. Prodromus Florae Peninsulae Balcanicae, vol. 2. Verlag des Repertoriums, Berlin.; Bothmer, R. von, 1970. Bot. Notiser 123: 52-60.; Greuter et al. 1986. Med-Checklist, vol. 3, Conservatoire et Jardin botaniques, Geneve.). Their main differences concern the leaf and calyx shape.

All chemotypes and genotypes of the species (a), (b) and (c) are included in the invention.

Typically, the extracts of the species (a), (b) and (c) are used in the following percentages by volume, based on the total volume of the extracts: (a) *Coridothymus capitatus:* 40-80%, (b) *Origanum dictamnus:* 3-15%, (c) *Salvia fruticosa* and/or *Salvia pomifera* 17-45%. Preferred are embodiments wherein the extract of species (a) is present in 50-75 %, the extract of species (b) is present in 5-12 % and/or the extract of species (c) is present in 20-38 %. More preferred are embodiments of the invention wherein the extract of species (a) is present in 57-65 %, the extract of species (b) is present in 7-9 % and/or the extract of species (c) is present in 28-34 %.

The botanical extracts of the species are preferably obtained by hydro-alcoholic extraction or hydro-distillation of the plant leaves. Hydro-alcoholic extraction is a known method of extracting essential oils from plant materials and typically involves the following steps:-
(i) The plant leaves are ground into small particles.
(ii) The ground matter is then mixed with aqueous ethanol (e.g. at a concentration of 90-95% ethanol) for a suitable time at a suitable temperature, e.g. in a Soxhlet apparatus. The liquid is separated from the solid residue, e.g. by filtration. This step may be repeated once or more, e.g. until the decoloration of the liquid before filtration. Different extraction volumes may be used; around 40 ml of ethanol per gram of ground leaves has been found to be suitable.
(iii) The filtered extract is concentrated by any suitable method, e.g. using a rotary evaporator.

Hydro-distillation is another known method of extracting essential oils from plant materials and typically involves the following steps:-
(i) The plant leaves are ground into small particles.
(ii) The ground material is then mixed with water for a suitable time at a suitable temperature e.g. in a Clenenger apparatus, according to the rules for the proper use of the apparatus.
(iii) The extract (distillate) is air-dried by any suitable method and it is then stored under refrigeration.

Hydro-alcoholic extraction and hydro-distillation procedure result in extracts having a slightly different composition, but both procedures are considered to result in an acceptable extract for the purposes of the invention.

The compositions of the present invention may act on the immune system, while their antioxidative effect has been well documented in previous experiments reported by one of the three inventors; see Lionis, et al., The Lancet 352: 1987-1988 (1998). Desirable objects of the pharmaceutical compositions of this invention are the treatment and/or prevention of influenza and coronavirus infection.

The term "treat" or "treatment" as used herein refers to any treatment of a disorder or disease related to microbial or viral infection in a subject and includes, for example, preventing the disorder or disease from occurring in a subject who has not yet been diagnosed as having the disorder or disease, inhibiting the disorder or disease, for example arresting the development of the disorder or disease, relieving the disorder or disease, for example, causing regression of the disorder or disease, or relieving the condition caused by the disease or disorder, for example, relieving the symptoms of the disorder or disease, and reducing the duration of the disorder.

Compositions of the present invention may be administered in any manner conventional for the treatment of the indicated diseases, including but not limited to oral, parenteral, sublingual, transdermal, rectal, or administration via inhalation or via buccal administration. Additionally, compositions of the present invention may be formulated for parenteral administration by injection or continuous infusion. The composition according to the invention may be formulated as a slow release form or as a depot preparation. The route of administration may be any route that effectively transports the active extracts to the desired site without harming the recipient.

The compositions of this invention may be prepared in conventional dosage unit forms by incorporating a mixture of the aforementioned extracts, with non-toxic pharmaceutical carrier diluent, excipient or adjuvant, according to accepted procedures, in a non-toxic amount sufficient to produce the desired pharmacological activity in a subject, animal or human. Preferably, the composition contains the active ingredients in an active, but non-toxic amount which depends on the specific biological activity desired and the condition of the patient.

The pharmaceutical carrier employed may be, for example, either a solid or a liquid. In the case of a solid carrier, the preparation can be plain milled, micronized or nanosized, in oil, tableted, placed in a hard gelatin or enteric-coated capsule in micronized powder or pellet form, or in the form of a troche, lozenge, or suppository. Representative solid carriers are lactose, terra alba, sucrose, talc, gelatin, agar, pectin, acacia, magnesium stearate, stearic acid, microcrystalline cellulose, polymer hydrogels and the like.

In the case of a liquid carrier, the preparation can be in the form of a liquid, such as an ampoule, or as an aqueous or non-aqueous liquid suspension mixed with pharmaceutically acceptable preservatives and the like. Typical liquid carriers are propylene glycol, aqueous solutions of β-cyclodextrins, syrup, peanut oil and olive oil and the like emulsions and solutions. Similarly, the carrier or diluent may include any time-delay material well known to the art, such as glycerol monostearate or glycerol distearate alone or with wax, microcapsules, microspheres, liposomes, and/or hydrogels.

In a preferred embodiment, the compositions are presented in soft-gel capsules, using olive oil (preferably extra virgin olive oil) as a carrier.

The amount of combined extract to be included in the unit dosage forms (e.g. capsules) corresponds to the amount of extract obtainable by hydro-alcoholic extraction or hydro-distillation from 0.2-3 g, preferably 0.5-1.7 g, more preferably 0.7-1.0 g of a mixture of dry plant material of the species (a), (b) and (c), when in the most preferred proportion of four parts by weight of dry material of species (a), half part by weight of dry material of species (b) and two parts by weight of dry material of species (c). In the case of hydro-distillation, the range of the amount of pure combined extract to be included in each unit dosage form is preferably 0.007-0.025 ml.

The typical proportion of the combined extract to carrier strongly depends on the carrier. In the case of extra virgin oil, it corresponds to the amount of combined extract to be included in the unit dosage in maximum one ml of extra virgin oil.

The recommended daily dose corresponds to one or two doses (in the unit dosage form). In the case of two doses per day the total duration of treatment may be reduced.

### EXPERIMENTAL SECTION

### Example 1

### Analysis of several chemotypes of the species (a), (b) and (c) (including microconstituents)

To ascertain the constituents of the essential oil of several chemotypes of the species used (a), (b) and (c), including microconstituents, analysis of plant extracts was performed by GC-MS.

**Table 1**

| | *Coridothymus capitatus* | *Origanum dictamnus* | *Salvia fruticosa* | *Salvia pomifera* |
|---|---|---|---|---|
| *trans*-2-hexenal | | + | | |
| α-Thujene | + | + | + | |
| α-Pinene | + | + | + | + |
| Camphene | + | + | + | + |
| Tricyclene | | | | + |
| β-Pinene | + | + | + | + |
| 1-Octen-3-ol | | + | | + |
| 3-Octanone | + | | | |
| Myrcene | + | + | + | + |
| 3-Octanol | + | + | | |
| α-Phellandrene | + | + | | |
| δ-3-carene | | + | | |
| *cis*-Dehydroxy linalool oxide | + | | | |
| α-Terpinene | + | + | | + |
| *p*-Cymene | + | + | | + |
| Limonene | + | + | | + |
| 1,8-Cineole | | | + | + |
| *trans*-β-ocimene | | + | | |
| β-Phellandrene | + | | | + |
| γ-Terpinene | + | + | + | + |
| *cis*-Sabinene hydrate | + | + | + | + |
| a-terpinolene | | | | + |
| Terpinolene | + | + | | |
| Linalool | + | + | | |
| a-Thujone | | | + | + |
| b-Thujone | | | + | + |
| iso-borneol | | + | | + |
| *trans*-Sabinene hydrate | + | + | | + |
| Camphor | | | + | + |
| *cis-p*-Menth-2-en-1-ol | + | | | |
| Bornyl acetate | | | | + |
| Borneol | + | + | + | |
| Terpinen-4-ol | + | + | | |
| α-Terpineol | + | | + | + |
| *cis*-Piperitol | + | | | |
| Thymol methyl ether | + | | | |
| *cis*-Dihydrocarvone | | + | | |
| *trans*-Dihydrocarvone | | + | | |
| Carvacrol methyl ether | + | + | | |
| 1-Carvone | | + | | |
| Thymoquinone | + | | | |
| Isobornyl acetate | + | | | |
| *p*-Cymen-7-ol | + | | | |
| Thymol | + | + | | |
| Carvacrol | + | + | | |
| Dihydrocarvyl acetate | | + | | |
| α-Cubebene | | + | | |
| *cis*-Carvyl acetate | | + | | |
| α-Copaene | | + | | |
| β-Bourbonene, | | + | | |
| β-Cubebene | | + | | |
| 4,8-alpha-epoxy caryophyllene | | + | | |
| Cubebol | | | | + |
| β-Caryophyllene | + | + | + | |
| α-Humulene | + | + | + | + |
| Epi-bicyclosesquiphellandrene | | + | | |
| Germacrene | + | | | |
| Germacrene D | | + | | |
| β-Bisabolene. | + | + | | |
| α-Amorphene | | + | | |
| α-Cadinene | | + | | |
| γ-Cadinene, | | + | | |
| δ-Cadinene | | + | | |
| Trimethylbicyclo-octan-2-one | | + | | |
| Caryophyllene oxide | | + | | + |
| T-cadinol | | + | | |
| Sabinene | | + | | + |
| Viridiflorol | | | + | |

### Example 2

### Investigation of the main essential components (% of the total essential oil) recorded in several chemotypes of Coridothymus capitatus.

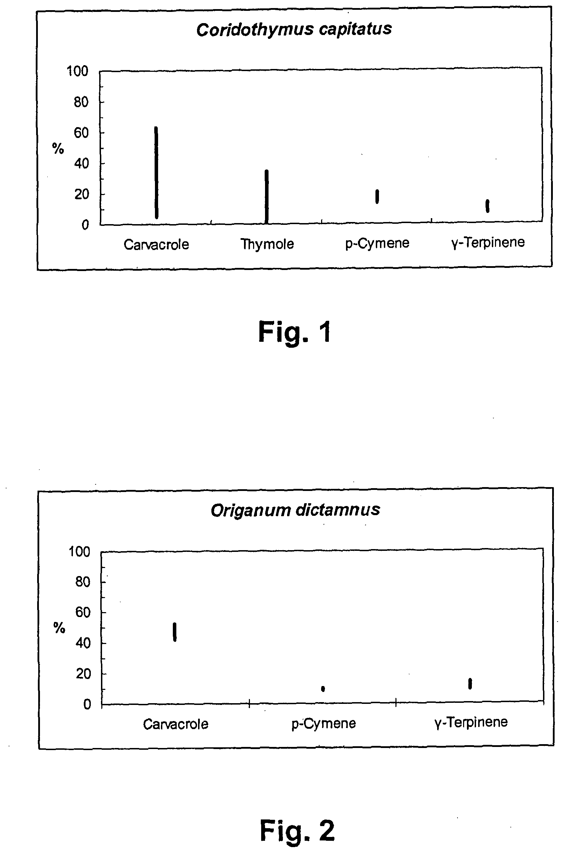

Analysis of 10 different preparations was performed by GC-MS. Plants collected in different periods of the year were included. Fig. 1 presents the range of each major constituent in % of the total essential oil in the hydro-distillate extract. The two highest values in the ranges correspond to the carvacrol and thymol content, while lower values correspond to the γ-terpinene and p-cymene content. In the case of carvacrol the range is from 5 to 62.6% and in the case of thymol from 0.3 to 34%.

### Example 3

### Investigation of the main essential components (% of the total essential oil) recorded in several chemotypes of Origanum dictamnus.

Analysis of 14 different preparations was performed by gas chromatography. Plants collected from different geographical areas in different periods of the year were included. Fig. 2 presents the percentage range of each major constituent in the hydro-distillate extract, as assayed by GC-MS. The range in all cases is narrow.

### Example 4

### Investigation of the main essential components (% of the total essential oil) recorded in several chemotypes of Salvia fruticosa.

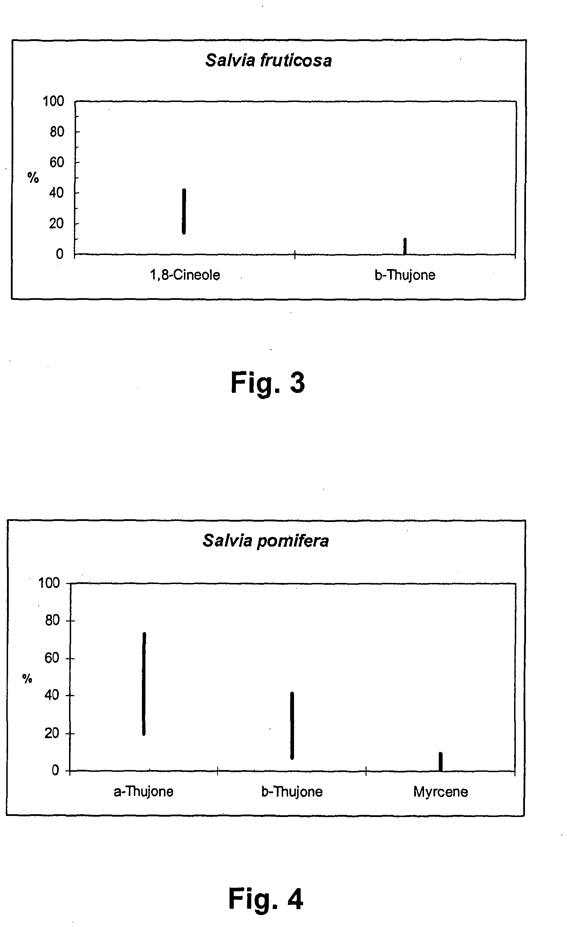

Analysis of 12 different preparations was performed by GC-MS. Plants collected from different geographical areas in different periods of the year were included. Fig. 3 presents the range of each major constituent in % of the total essential oil in the hydro-distillate extract. The range of values of 1,8-cineole content was higher than the corresponding values for b-thujone.

### Example 5

### Investigation of the main essential components (% of the total essential oil) recorded in several chemotypes of Salvia pomifera.

Analysis of 9 different preparations was performed by GC-MS. Plants collected from different geographical areas in different periods of the year were included. Fig. 4 presents the range of each major constituent in % of the total essential oil in the hydro-distillate extract. The range of values for a- and b-thujone was higher than the corresponding values for myrcene.

### Example 6

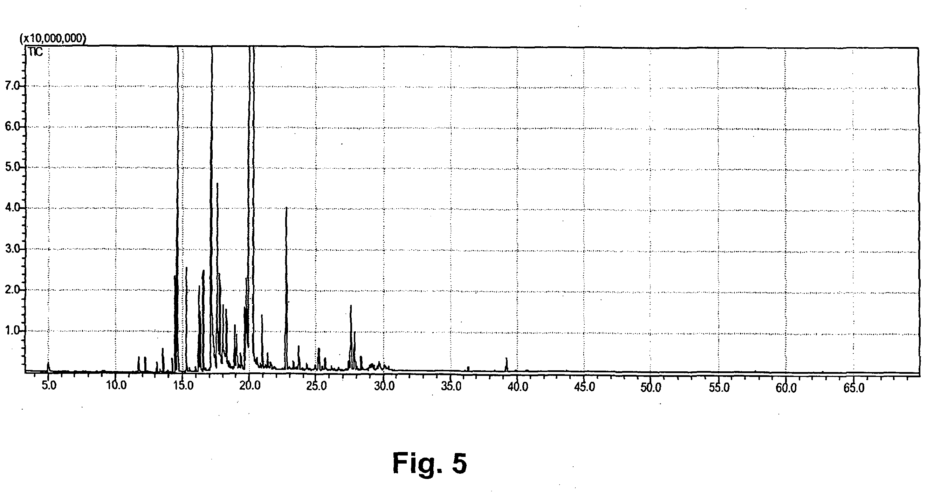

Fig. 5 shows a typical gas chromatography trace of a hydrodistillate combined extract of the preferred composition according to the invention. The major identified compounds are presented in Table 2. The identification of the main components was based a) on the Retention Time of each component in relation to the Retention Time of reference compounds, b) the Wiley library Spectra and NIST/NBS, c) literature data (Massada, 1976, Analysis of Essential Oils by Gas chromatography and Mass Spectrometry, Wiley, New York, Adams, 2001; Identification of Essential Oil Components by Gas Chromatography/Quadrupole Mass Spectroscopy, Allured Publ. Corp., Carol Stream, IL, Bicchi and Joulain, 1990, Flavour and Fragrance Journal 5: 131-145, Bruneton, 1993, Pharmacognosie, Phytochimie, Plantes Médicinales. 2nd edn, Ed, Tec & Roc, Paris) and d) Kovàts indexes (Van den Dool and Kratz, 1963 Journal of Chromatography 11: 463-471.

**Table 2**

| Essential oil composition (% of the total essential oil) | | |
|---|---|---|
| **No** | **Compound** | **%** |
| 1 | α- Thujene | 0.02 |
| 2 | α-Pinene | 0.24 |
| 3 | Camphene | 0.24 |
| 4 | β-Pinene | 0.13 |
| 5 | 1-Octen-3-ol | 0.05 |
| 6 | β-Myrcene | 0.27 |
| 7 | α-Phellandrene | 0.03 |
| 8 | 3-Carene | 0.01 |
| 9 | α-Terpinene | 0.17 |
| 10 | p-Cymene | 1.47 |
| 11 | Eucalyptol | 9.68 |
| 12 | γ-Terpinene | 1.16 |
| 13 | *cis*-Sabinene Hydrate | 0.05 |
| 14 | Terpinolene | 0.07 |
| 15 | Linalool | 1.10 |
| 16 | *cis*-Thujone | 0.85 |
| 17 | *trans*-Thujone | 1.25 |
| 18 | Camphor | 8.93 |
| 19 | *trans*-Pinocamphone | 0.18 |
| 20 | Borneol | 3.31 |
| 21 | Terpinen-4-ol | 1.42 |
| 22 | α-Terpineol | 0.93 |
| 23 | Terpineol | 0.94 |
| 24 | Verbenone | 0.08 |
| 25 | 1,3,3,4-Tetramethyl-2-oxabicyclo[2.2.0]hexane | 0.10 |
| 26 | Thymol methyl ether | 0.53 |
| 27 | Verbenone | 0.72 |
| 28 | β-Citral | 0.04 |
| 29 | Carvone | 0.28 |
| 30 | Bornyl acetate | 0.89 |
| 31 | Thymol | 2.32 |
| 32 | Carvacrol | 54.13 |
| 33 | α-Terpineol acetate | 0.75 |
| 34 | Eugenol | 0.04 |
| 35 | Thymol acetate | 0.24 |
| 36 | Neryl acetate | 0.11 |
| 37 | α-Copaene | 0.10 |
| 38 | β-Bourbonene | 0.03 |
| 39 | α-Gurjunene | 0.02 |
| 40 | β-Caryophyllene | 3.06 |
| 41 | Aromadendrene | 0.15 |
| 42 | α-Humulene | 0.47 |
| 43 | γ-Muurolene | 0.10 |
| 44 | α-Bisabolene | 0.26 |
| 45 | γ-Cadinene | 0.12 |
| 46 | δ-Cadinene | 0.27 |
| 47 | Spathulenol | 0.24 |
| 48 | Caryophyllene oxide | 1.29 |
| 49 | Epiglobulol | 0.80 |
| 50 | Cubenol | 0.03 |
| 51 | tau.Cadinol | 0.05 |
| 52 | Isoaromadendrene epoxide | 0.28 |
| | TOTAL | 100 |

### Example 7

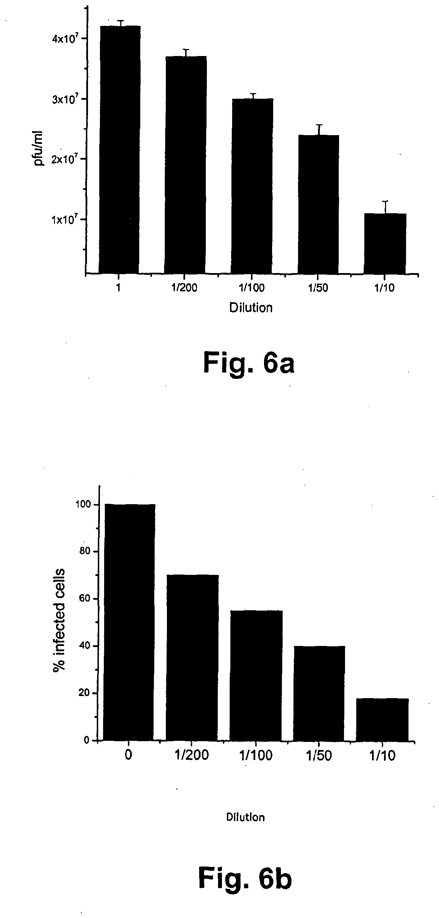

Fig. 6a provides an example of the anti-viral capacity of a preferred extract mixture of the invention, showing the inhibition of Herpes Simplex Virus (HSV) plaque formation units (pfu) per ml of a sample of transfected cells. The effect of a preferred extract mixture of the invention on Herpes Simplex Virus type-1 (HSV-1) was initially determined by plaque assay according to standard protocols. Briefly, BHK cells were infected by wild-type HSV-1 at a multiplicity of infection (M.O.I.) 1 and treated, in parallel, with various dilutions of the extract mixture ranging from 1 to 1/200. The cells were incubated for 24 hours, allowing sufficient time for the virus to complete its life cycle. Subsequently, the supernatants from all wells were passed on fresh BHK cells for 1 hour and then the inoculums were removed and replaced with fresh medium containing anti-HSV-1 human serum. The number of viral plaques was counted 48 hours post infection, representing the number of viral plaque forming units per milliliter (pfu/ml).

Fig. 6b presents a bar chart of the % of viral infected cells in the presence of a preferred extract preparation of the invention. The anti-viral effect was quantified by indirect immunofluorescence, after the passage of the above supernatant on BHK cells and staining for a late HSV-1 protein (gG) 24 hours post-infection. In each treatment (dilution), the number of infected cells was compared to the total number of cells and also relatively to the number of infected cells in the absence of the compound.

### Example 8

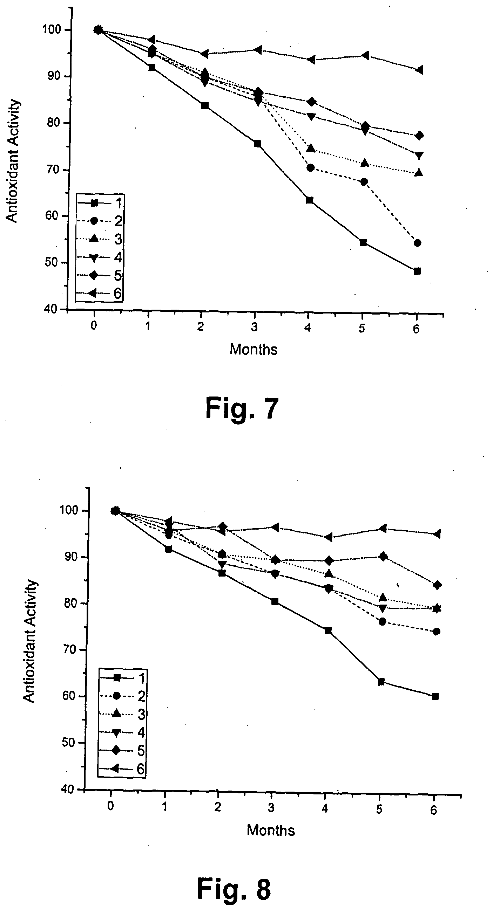

The stability of a hydro-alcoholic preparation of the most preferred combined extract was investigated in different experimental conditions. The Folin-Ciocalteu assay was used for the analysis. The results are presented in Fig. 7 as a percentage value of the initial phenolic concentration of the preparation. The test conditions were as follows:
- 1: Room Temperature, Air, Ambient light
- 2: Room Temperature, Air, Dark
- 3: 4°C, Air, Dark
- 4: Room Temperature, Sealed, Ambient Light
- 5: Room Temperature, Sealed, Dark
- 6: 4°C, Sealed, Dark

### Example 9

The stability was also investigated of the same extract in an olive oil vehicle. The Folin-Ciocalteu assay was used for the analysis. The results are presented in Fig. 8 as a percentage value of the initial phenolic concentration of the preparation. Vehicle values were systematically substracted. The test conditions were as follows:
- 1: Room Temperature, Air, Ambient light
- 2: Room Temperature, Air, Dark
- 3: 4°C, Air, Dark
- 4: Room Temperature, Sealed, Ambient Light
- 5: Room Temperature, Sealed, Dark
- 6: 4°C, Sealed, Dark

## Claims

1. A composition comprising effective amounts of extracts of:
(a) *Coridothymus capitatus*;
(b) *Origanum dictamnus*; and
(c) at least one of *Salvia fruticosa* or *Salvia pomifera.*

2. A composition as claimed in claim 1 wherein the said extracts are obtainable by hydro-alcoholic extraction of the plant leaves or hydro-distillation of the plant leaves.

3. A composition as claimed in any preceding claim further comprising one or more pharmaceutically acceptable carrier, diluent, excipient or adjuvant.

4. A composition as claimed in any preceding claim wherein the extracts are present in the following percentages by volume, based on the total volume of the extracts of the species (a), (b) and (c): 40-80% (a), 3-15% (b) and 17-45% (c).

5. A composition as claimed in claim 4, comprising 50-75% of the extract of the species (a), based on the total volume of the extracts of the species (a), (b) and (c).

6. A composition as claimed in any preceding claim comprising 5-12% of the extract of the species (b), based on the total volume of the extracts of the species (a), (b) and (c).

7. A composition as claimed in any preceding claim comprising 20-38% of the extract of the species (c), based on the total volume of the extracts of the species (a), (b) and (c).

8. A composition as claimed in any of claims 1 to 3 wherein the extracts of the species (a), (b) and (c) are present in the ratio of four parts by volume of the extract of species (a), two parts by volume of the extract of species (c) and half part by volume of the extract of species (b).

9. A combination of the extracts of species (a), (b) and (c) as defined in claim 1, for use in therapy.

10. A combination of the extracts of species (a), (b) and (c) as defined in claim 1, for use in preventing or treating influenza or coronavirus infection.

11. A combination of the extracts of species (a), (b) and (c) as defined in claim 1, for use in preventing or treating influenza type A/H1N1 species.

12. Use of the extracts of species (a), (b) and (c) as defined in claim 1, for the manufacture of a medicament for preventing or treating influenza or coronavirus infection.

13. A process comprising the following steps:
1) providing extracts of species (a), (b) and (c) as defined in claim 1; and
2) mixing, in any order, the extracts and optionally one or more pharmaceutically acceptable carrier, diluent, excipient or adjuvant.

14. A process as claimed in claim 13 wherein the extracts are obtained by hydro-alcoholic extraction of the plant leaves or by hydro-distillation of the plant leaves.

## Patentansprüche

1. Zusammensetzung, umfassend wirksame Mengen von Extrakten von:
(a) *Coridothymus capitatus*;
(b) *Origanum dictamnus*; und
(c) mindestens einem von *Salvia fruticosa* oder *Salvia pomifera.*

2. Zusammensetzung nach Anspruch 1, wobei die Extrakte durch hydroalkoholische Extraktion der Pflanzenblätter oder Hydrodestillation der Pflanzenblätter erhältlich sind.

3. Zusammensetzung nach einem der vorstehenden Ansprüche, weiter umfassend einen oder mehrere pharmazeutisch akzeptable Träger, Verdünnungsmittel, Hilfsstoffe oder Adjuvantien.

4. Zusammensetzung nach einem der vorstehenden Ansprüche, wobei die Extrakte in den folgenden Volumenprozenten vorhanden sind, basierend auf dem Gesamtvolumen der Extrakte der Spezies (a), (b) und (c): 40-80% (a), 3-15% (b) und 17-45% (c).

5. Zusammensetzung nach Anspruch 4, umfassend 50-75% des Extrakts der Spezies (a), basierend auf dem Gesamtvolumen der Extrakte der Spezies (a), (b) und (c).

6. Zusammensetzung nach einem der vorstehenden Ansprüche, umfassend 5-12% des Extrakts der Spezies (b), basierend auf dem Gesamtvolumen der Extrakte der Spezies (a), (b) und (c).

7. Zusammensetzung nach einem der vorstehenden Ansprüche, umfassend 20-38% des Extrakts der Spezies (c), basierend auf dem Gesamtvolumen der Extrakte der Spezies (a), (b) und (c).

8. Zusammensetzung nach einem der Ansprüche 1 bis 3, wobei die Extrakte der Spezies (a), (b) und (c) im Verhältnis von vier Volumenteilen des Extrakts von Spezies (a), zwei Volumenteilen des Extrakts von Spezies (c) und einem halben Volumenteil des Extrakts von Spezies (b) vorhanden sind.

9. Kombination der Extrakte von Spezies (a), (b) und (c), wie in Anspruch 1 definiert, zur Verwendung in einer Therapie.

10. Kombination der Extrakte von Spezies (a), (b) und (c), wie in Anspruch 1 definiert, zur Verwendung bei der Prävention oder Behandlung einer Influenza- oder Coronavirus-Infektion.

11. Kombination der Extrakte von Spezies (a), (b) und (c), wie in Anspruch 1 definiert, zur Verwendung bei der Prävention oder Behandlung von Influenza-Typ-A/H1N1-Spezies.

12. Verwendung der Extrakte von Spezies (a), (b) und (c), wie in Anspruch 1 definiert, zur Herstellung eines Medikaments zur Prävention oder Behandlung einer Influenza-oder Coronavirus-Infektion.

13. Verfahren, umfassend die folgenden Schritte:
1) Bereitstellen von Extrakten von Spezies (a), (b) und (c), wie in Anspruch 1 definiert; und
2) Mischen, in einer beliebigen Reihenfolge, der Extrakte und wahlweise eines oder mehrerer pharmazeutisch akzeptabler Träger, Verdünnungsmittel, Hilfsstoffe oder Adjuvantien.

14. Verfahren nach Anspruch 13, wobei die Extrakte durch hydroalkoholische Extraktion der Pflanzenblätter oder durch Hydrodestillation der Pflanzenblätter erhalten werden.

## Revendications

1. Composition comprenant des quantités efficaces d'extraits de :
(a) *Coridothymus capitatus* ;
(b) *Origanum dictamnus* ; et
(c) au moins une de *Salvia fruticosa* ou *Salvia pomifera.*

2. Composition selon la revendication 1, dans laquelle lesdits extraits peuvent être obtenus par extraction hydroalcoolique des feuilles de la plante ou par hydrodistillation des feuilles de la plante.

3. Composition selon l'une quelconque des revendications précédentes, comprenant en outre un ou plusieurs vecteurs, diluants, excipients ou adjuvants acceptables d'un point de vue pharmaceutique.

4. Composition selon l'une quelconque des revendications précédentes, dans laquelle les extraits sont présents dans les pourcentages suivants en volume, sur la base du volume total des extraits des espèces (a), (b) et (c) : 40 à 80 % (a), 3 à 15 % (b) et 17 à 45 % (c).

5. Composition selon la revendication 4, comprenant 50 à 75 % de l'extrait de l'espèce (a), sur la base du volume total des extraits des espèces (a), (b) et (c).

6. Composition selon l'une quelconque des revendications précédentes, comprenant 5 à 12 % de l'extrait de l'espèce (b), sur la base du volume total des extraits des espèces (a), (b) et (c).

7. Composition selon l'une quelconque des revendications précédentes, comprenant 20 à 38 % de l'extrait de l'espèce (c), sur la base du volume total des extraits des espèces (a), (b) et (c).

8. Composition selon l'une quelconque des revendications 1 à 3, dans laquelle les extraits des espèces (a), (b) et (c) sont présents dans le rapport de quatre parties en volume de l'extrait de l'espèce (a), deux parties en volume de l'extrait de l'espèce (c) et une demi-partie en volume de l'extrait de l'espèce (b).

9. Combinaison des extraits des espèces (a), (b) et (c) selon la revendication 1, pour une utilisation en thérapie.

10. Combinaison des extraits des espèces (a), (b) et (c) selon la revendication 1, pour une utilisation dans la prévention ou le traitement d'une grippe ou d'une infection par coronavirus.

11. Combinaison des extraits des espèces (a), (b) et (c) selon la revendication 1, pour une utilisation dans la prévention ou le traitement de la grippe du type A/H1N1.

12. Utilisation des extraits des espèces (a), (b) et (c) selon la revendication 1, pour la fabrication d'un médicament pour empêcher ou traiter une grippe ou une infection par coronavirus.

13. Processus comprenant les étapes suivantes :
1) fourniture d'extraits des espèces (a), (b) et (c) selon la revendication 1 ; et
2) mélange, dans n'importe quel ordre, d'extraits et de manière facultative d'un ou plusieurs vecteurs, diluants, excipients ou adjuvants acceptables d'un point de vue pharmaceutique.

14. Processus selon la revendication 13, dans lequel les extraits sont obtenus par extraction hydroalcoolique des feuilles de la plante ou par hydrodistillation des feuilles de la plante.
